# DEMANDE DE BREVET EUROPEEN

(11) **EP 1 870 423 A1**
(43) Date de publication de la demande: **26.12.2007**
(21) Numéro de dépôt: 06370021.5
(22) Date de dépôt: 22.06.2006
(51) Int. Cl.: C07K 16/28, C07K 14/705, C12N 5/00, G01N 33/543, G01N 33/569

(54) **Structure antigénique de surface de cellules spermatique associée au chromosome Y**

(71) Demandeur: Genes Diffusion, 59500 Douai (FR)
(72) Inventeur: Duban-Deweer, Sophie, 59650 Villeneuve d'Asq (FR); Liégois, Luc, 59283 Raimbeaucourt (FR)
(74) Mandataire: Matkowska, Franck

(57) **Abrégé**

La présente invention est relative à une structure antigénique de surface de cellules spermatiques et associées au chromosome Y, aux molécules, en particulier des anticorps dirigés contre cette structure antigénique et à une méthode permettant de caractériser les cellules portant uniquement le chromosome Y par l'interaction entre cette structure antigénique et les molécules dirigées contre celle-ci.

## Description

### Objet de l'invention

La présente invention est relative à une structure antigénique présente sur des cellules spermatiques (spermatozoïdes) et associées au chromosome Y et des molécules, en particulier des anticorps ou des portions de ceux-ci, dirigés contre cette structure antigénique.

La présente invention est aussi relative à un procédé permettant de caractériser la présence du chromosome y dans une cellule, en particulier dans une cellule spermatique (spermatozoïde).

L'invention est également relative à une méthode permettant de caractériser le sexe de la descendance de mammifères, y compris l'humain, par une séparation de cellules de spermatozoïdes déterminées pour être mâles et portant le chromosome Y, et basée sur l'utilisation de l'interaction de cette structure antigénique avec des molécules de préférence marquées, en particulier des anticorps marquées, dirigés contre celle-ci.

### Arrière-plan technologique et état de la technique à la base de l'invention

Dans des conditions naturelles de croissement et d'insémination artificielle (IA) le ratio de genre d'une descendance est approximativement de 50% de mâles (XY) et de 50% de femelles (XX).

Les fermiers et autres éleveurs d'animaux expriment depuis longtemps leur désir d'augmenter la probabilité de réaliser une descendance de sexes sélectionnés (de préférence par la sélection de cellules spermatozoiques porteurs du chromosome Y ou porteurs du chromosome X avant une insémination des mammifères).

Au-delà des aspects physiologiques évidents, la sélection de la descendance en fonction du sexe a des conséquences économiques significatives, si l'on considère ces applications à la production de nourriture animale (pour les caprins, les bovins et les porcins), mais également ces applications au niveau des concours d'animaux (en particulier des chevaux et des chiens).

Différentes directions technologiques ont été prises afin d'arriver à sélectionner le genre dans les mammifères, aussi bien pour l'intérêt zootechnique des espèces, que pour les espèces en voie d'extinction, mais aussi pour les animaux domestiques et pour la reproduction assistée pour l'humain. Il y a deux alternatives pour cela : séparer les cellules spermatiques portant le chromosome X de celles portant le chromosome Y, ou déterminer le sexe d'un embryon avant réimplantation.

Chez l'humain, la sélection du sexe d'un embryon implique de fortes contraintes éthiques qui peuvent être éliminées par le sexage. Le sexage des cellules spermatiques pourrait également avoir des applications cliniques afin de prévenir des maladies liées au chromosome X dans l'espèce humaine, telle que la dystrophie musculaire de Duchenne, l'hémophilie A ou B, la dépigmentation rétinienne, la maladie de Lesh Nyan ou syndrome du X fragile, conduisant à des retards mentaux (ZARUTSKIE et al. 1989). Il y a environ 6000 anomalies génétiques et plus de 370 d'entre elles sont liées au chromosome X (COTINOT et al., 1993, JOHNSON et al. 1993).

Différents efforts ont été menés afin d'aboutir à la sélection de la descendance sur le sexe. Ceux qui apparaissent les plus intéressants pour l'aboutissement du résultat désiré ont été orientés vers la sortie et la sélection de cellules de sperme X ou Y.

Toute technique de séparation, pour être efficace, doit utiliser des différences phénotypiques entre les cellules de sperme X et Y. La séparation des spermatozoïdes portant le chromosome X, de ceux portant le chromosome Y est liée à la détection d'au moins une différence entre ces phénotypes. Dans ce contexte, des essais de séparation ont été faits sur la base de différences apparentes (chimiques ou physiques) variées entre les cellules de spermatozoïdes X et Y (telle qu'une différence de fluorescence, de taille, de masse, de densité, de vitesse de nage, de sensibilité au pH, d'adhérence au séphadex, etc).

Des moyens de séparation immunologique des spermatozoïdes portant le chromosome Y, des spermatozoïdes portant le chromosome X ont également été suggérés.

Le brevet US 4 448 767 décrit une méthode qui nécessite le développement d'anticorps contre les marqueurs de la surface cellulaire spécifique capables de discriminer les cellules X des cellules Y.

Un antigène H-Y a été utilisé comme marqueur pour la séparation des cellules spermatiques porteurs du chromosome X ou du chromosome Y (Ali et al., 1990 ; Peter et al. 1993). Cependant, d'autres chercheurs ont rapporté leur incapacité à isoler des cellules de type X ou Y en utilisant le marqueur antigénique H-Y (Hendriksen et al. 1993 ; Sins et al. 1998). Le consensus actuel est que l'antigène H-Y ne permet pas une séparation entre des cellules portant le chromosome X ou le chromosome Y.

Le brevet US 5 660 997 décrit l'utilisation de protéines membranaires liées au sexe (protéines SAM). Ces protéines SAM étant caractérisées par un poids moléculaire particulier déterminé sur gel SDS polyacrylamide (PAGE) et par un point isoélectrique (PI), déterminé par une immobilisation sur un gel à gradient de pH (IPG). Chez l'espèce de chiens Chinchilla, les protéines SAM -X ont des poids moléculaires de respectivement 19 kDa, 25 kDa, 29 kDa, 32 kDa, 39 kDa, 72 kDa et 120 kDa tandis que les protéines SAM du chromosome Y ont des poids moléculaires de 15 kDa, 45 kDa, 55 kDa, 64 kDa et 125 kDa.

Cependant, cette demande de brevet ne donne pas de données d'efficacité de la méthode et la preuve que ces protéines caractérisées sont effectivement liées au chromosome X ou Y des cellules et que ladite liaison ait pour conséquence la déviation significative du sexe - ratio dans les espèces de mammifères

Le brevet US 5 021 244 décrit un anticorps qui se fixe de manière spécifique sur les membranes de cellules portant le chromosome X (X-SAM). Ledit anticorps (de préférence monoclonal) est essentiellement dépourvu d'anticorps capables de fixer des protéines membranaires associées au chromosome Y ou associées aux antigènes H-Y, ou à des composants non spécifiques du sexe présents dans la membrane plasmatique de la cellule.

### Buts de l'invention

La présente invention vise à fournir une nouvelle structure antigénique de surface de cellules spermatiques, associée au chromosome Y et permettant d'obtenir une discrimination entre des cellules spermatiques (spermatozoïdes) portant le chromosome Y et des cellules portant le chromosome X.

La présente invention vise à fournir également des molécules de préférence marquées, en particulier des anticorps marquées, tels que des anticorps monoclonaux ou des portions de ceux-ci, dirigés spécifiquement contre cette structure antigénique et d'utiliser l'interaction entre cette structure antigénique et ces molécules pour obtenir un marquage spécifique de ces cellules, et aussi afin de pouvoir discriminer et éventuellement séparer des cellules portant uniquement le chromosome Y des autres cellules (portant le chromosome X et/ou éventuellement portant le chromosome Y).

La présente invention est également relative à un procédé d'insémination de mammifères par lesdites cellules discriminés.

### Résumé de l'invention

La présente invention est relative à une structure antigénique de surface de cellules spermatiques (spermatozoïdes) spécifique du chromosome Y et ayant une masse moléculaire inférieure à 15 kDa, de préférence inférieure à 10 kDa, plus particulièrement présentant une masse moléculaire comprise entre 5 et 10 kDa, de préférence une masse moléculaire respectivement d'environ 5 kDa, 7 kDa et 10 kDa. La spécificité de cette structure antigénique peut être déterminé par un western blot marquant spécifiquement et uniquement cette structure antigénique d'intérêt présente uniquement sur le chromosome Y.

Par conséquent, un premier objet de l'invention est relatif à 1 utilisation de la structure antigénique d'intérêt de l'invention pour le marquage spécifique de cellules exprimant le chromosome Y par rapport à des cellules n'exprimant pas ce chromosome Y (cellules comportant le chromosome x uniquement).

Cette analyse peu s'effectuer sur des cellules vivantes par un marquage de la surface de la cellule ou sur des extraits cellulaires, par exemple par un western blot.

Un autre aspect de la présente invention est relatif à des molécules, de préférence marquées et dirigées contre cette structure antigénique.

Ces molécules sont de préférence des anticorps marquées ou des portions hypervariables de ces anticorps dirigées spécifiquement contre cette structure antigénique .Ces molécules peuvent être également des nanobodies™ qui sont une nouvelle classe de protéines thérapeutique dérivées d'anticorps. Ces exemples de molécules sont dérivés d'anticorps de la famille des camelidae qui sont dépourvus de la chaîne légère spécifique des anticorps mais qui conserve une capacité complète à fixer des antigènes. Il est donc possible d'obtenir des nanobodies constitués uniquement de la portion du domaine variable de l'extrémité d'un anticorps (www.ablynx.com).

De préférence, l'anticorps est un anticorps monoclonal.

Un autre aspect de la présente invention concerne la cellule (hybridome) apte à produire ces anticorps ou les portions hypervariables de ces anticorps.

La présente invention est également relative à une trousse de diagnostic (de sexage) comprenant lesdites molécules (éventuellement marquées) dirigées spécifiquement contre cette structure antigénique, en particulier lesdits anticorps ou les portions hypervariables de ceux-ci, ainsi que tous les milieux et les réactifs destinés à caractériser l'interaction entre ces structures antigéniques et lesdites molécules dirigées spécifiquement contre celles-ci.

Ces milieux et réactifs sont par exemple des solutions de lavage et des éléments de marquage desdites molécules dirigées contre la structure antigénique (tels que des marqueurs colorimétriques, luminescents, chemoluminescents, bioluminescents, fluorescents ou radioactifs). Ces milieux ou réactif peuvent également comporter des supports solides aptes à interagir avec les dites molécules dirigées contre la structure antigénique. Des supports solides aptes à interagir avec ces molécules sont des billes, des billes d'agarose ou des billes métalliques (en particulier des billes magnétiques), d'une taille adéquate (entre 50 nm et 4 µm) de manière à pouvoir fixer et marquer efficacement des cellules vivantes et à obtenir ensuite une séparation de cellules spermatiques portant spécifiquement le chromosome Y à partir d'un mélange de cellules spermatiques.

La trousse de l'invention peut également comporter des moyens de certification des caractéristiques sexuelles des chromosomes portés par lesdits spermatozoïdes discriminés. Ces moyens sont constitués par une trousse d'amplification génétique de gènes spécifiques, soit du chromosome X, soit du chromosome Y. Ces moyens sont de préférence constitués par des amorces, des enzymes et des milieux réactionnels permettant une amplification génétique par une méthode choisie parmi le groupe constitué par la PCR, la LCR, ou le NASBA.

La trousse de l'invention peut être également utilisée pour confirmer si d'autres moyens de séparation (par des procédés physiques, chimiques ou biotechnologiques) de cellules sont efficaces. La trousse de l'invention peut donc être utilisée comme moyen de certification de la présence du chromosome Y sur les cellules ou dans des extraits cellulaires.

Un autre aspect de la présente invention concerne un support solide comprenant lesdites molécules dirigées spécifiquement contre la structure antigénique de l'invention, les molécules étant fixées sur la surface du support solide, de manière directe ou indirecte (via une interaction par un couple de molécules, telles que biotines et des streptavidines ou des avidines) et aptes à fixer spécifiquement des cellules spermatiques comportant le chromosome Y, via l'interaction entre la structure antigénique et lesdites molécules

Le support solide peut, par exemple, être constitué du support solide d'une colonne de chromatographie, de plaques de multi-puits, de billes (dont la taille est de préférence comprise entre 50 nm et 4 µm, de préférence entre 100 et 400 nanomètres) en particulier de billes magnétiques, permettant une interaction entre la structure antigénique des cellules et les molécules dirigées contre ceux-ci, permettant une discrimination et, avantageusement, permettant une séparation des différentes catégories de cellules (séparation entre les cellules portant uniquement le chromosome Y et entre les autres cellules (cellules portant le chromosome X et éventuellement d'autre cellules portant le chromosome Y).

La présente invention est également relative à un procédé de discrimination et éventuellement de séparation entre des cellules portant le chromosome Y ou le chromosome X par l'utilisation de l'interaction entre cette structure antigénique et ces molécules, en particulier ces anticorps ou des portions de ceux-ci dirigés contre cette structure antigénique.

Le procédé de l'invention comprend les étapes suivantes :
- une mise en contact des cellules spermatiques avec les molécules ou le support solide de l'invention (comprenant lesdites molécules) dirigées spécifiquement contre la structure antigénique de l'invention qui est spécifique du chromosome Y et présente à la surface desdites cellules, dans des conditions aptes à obtenir une interaction (liaison ou marquage) spécifique entre ladite structure antigénique et lesdites molécules ;
- une liaison ou un marquage des cellules portant le chromosome Y par l'interaction entre lesdites molécules et ladite structure antigénique, ledit marquage étant obtenu par un signal résultant de l'interaction entre ladite structure antigénique présente à la surface des cellules et lesdites molécules dirigées contre celles-ci,
- éventuellement une séparation entre les cellules liées ou marquées par lesdites molécules et les cellules non liées et/ou non marquées par les dites molécule,
- éventuellement recueillement soit des cellules liées ou marquées, soit des autres cellules (non liées et non marquées et portant majoritairement le chromosome X), et
- éventuellement insémination d'un mammifère par lesdites cellules (insémination d'un mammifère par lesdites cellules recueillies et portant le chromosome Y uniquement ou insémination des mammifères par les autres cellules recueillies et portant majoritairement le chromosome X).

Selon l'invention, les molécules dirigées contre la structure antigénique sont des anticorps (de préférence monoclonaux) ou une portion hypervariable de ceux-ci. Ces anticorps pouvant être marqués directement ou indirectement par un élément de marquage colorimétrique, luminescent, chemoluminescents, fluorescent ou radioactif. Ce marquage peut être un marquage direct ou indirect.

On entend par marquage direct, une fixation covalente du marqueur (colorimétrique, luminescent, fluorescent, radioactif) sur ladite molécule, de préférence sur ledit anticorps.

On entend par marquage indirect, une liaison de la première molécule avec une seconde molécule déjà liée à ce marqueur. Par exemple on peut faire réagir la première molécule, (étant un anticorps), avec une seconde molécule (un second anticorps) dirigée spécifiquement contre la partie constante du premier anticorps, le second anticorps étant lié covalement au marqueur colorimétrique, luminescent, chemoluminescents, fluorescent ou radioactif, ou le support solide (billes).

Dans le procédé de l'invention, la caractérisation des cellules est obtenue par une mise en contact des molécules de l'invention avec les cellules. Cette opération se fait dans des milieux permettant la survie desdites cellules, mais permettant également d'éliminer toute interférence avec des composants susceptibles d'interférer avec l'interaction entre les cellules et lesdites molécules dirigées contre la structure antigénique des cellules.

L'étape de séparation et de recueillement entre des cellules portant le chromosome Y uniquement et les autres cellules s'effectue grâce au marquage des cellules par les molécules interagissant avec la structure antigénique. De préférence, cette séparation peut se faire sur un support solide (de préférence, des billes magnétiques) sur lequel lesdites molécules ont été fixées et avec lesquelles les cellules sont susceptibles d'entrer en interaction.

Un milieu permettant l'élution des autres cellules non liées ou non marquées permet d'obtenir une séparation efficace. Cette étape de séparation et éventuellement de recueillement des cellules liées ou marquées, est connue de l'homme de l'art.

L'étape d'insémination de mammifères par des cellules comportant uniquement le chromosome Y et/ou éventuellement de mammifères par du sperme comprenant majoritairement des cellules portant uniquement le chromosome X (et éventuellement une plus faible proportion de cellules portant le chromosome Y), s'effectue également par des méthodes bien connues de l'homme de l'art.

On entend par les termes « du sperme comprenant majoritairement des cellules portant uniquement le chromosome X », le fait qu'une proportion de cellules supérieure à 50%, de préférence supérieure à 75%, plus particulièrement supérieure à 90% ou 95%, portent uniquement le chromosome X.

Par ces étapes d'insémination, il est possible d'obtenir des descendances uniquement masculines ou des descendances essentiellement féminines, de préférence uniquement féminines.

Les mammifères inséminables sont de préférence des mammifères importants pour une production de nourriture animale, tels que les caprins, les bovins et les porcins, (de préférence des bovins), mais peuvent également être des animaux domestiques, tels que des chevaux, des lapins, des chiens ou des chats ou des mammifères appartenant à des espèces en voie d'extinction. Les mammifères peuvent être également des primates, y compris l'humain, notamment des sujets dont les parents sont porteurs de maladies héréditaires liés au chromosome X, telle que la dystrophie musculaire de Duchenne, l'hémophilie A ou B, la dépigmentation rétinienne, la maladie de Lesh Nyan ou le syndrome du X fragile, conduisant à des retards mentaux.

Le procédé de l'invention peut être éventuellement combiné à d'autres procédés de séparation des cellules portant le chromosome Y ou le chromosome X, par exemple des méthodes de séparation basées sur des différences chimiques ou physiques associées aux cellules portant le chromosome X ou aux cellules portant le chromosome Y, telles que des différences de fluorescence, de taille, de masse, de densité, de vitesse de nage, de sensibilité au pH, d'adhérence au séphadex, etc.

Les différentes étapes du procédé de l'invention peuvent être éventuellement combinées à une étape de caractérisation spécifique du sexe des cellules effectuée sur la totalité des cellules sélectionnées ou sur un échantillon de ceux-ci, de manière à assurer que ces étapes sont effectuées de manière optimale.

Ce procédé de caractérisation peut par exemple consister en une étape ou plusieurs étapes d'amplification génétique (par PCR, LCR, NASBA, ... d'un ou plusieurs marqueurs spécifiques du chromosome X ou du chromosome Y.

La présente invention sera décrite de manière plus détaillée dans l'exemple d'exécution ci-dessous, présenté à titre d'illustration non limitative du procédé de l'invention en référence aux figures annexées.

### Brève description des figures

La figure 1 représente l'analyse du protéome total des cellules permettant d'isoler une fraction protéique (Fraction F) spécifique des cellules de type Y.

La figure 2 représente les trois isoformes de cette fraction protéique caractérisés par leur poids moléculaire sur un gel d'électrophorèse.

La figure 3 représente les marquages d'immunolocalisation *in vivo* de cellules spermatiques réalisés avec l'anticorps de l'invention. Les cellules marquées et non-marquées sont représentées sur la figure 3.

### Description détaillée de l'invention

### 1.Obtention d'une fraction protéique spécifique

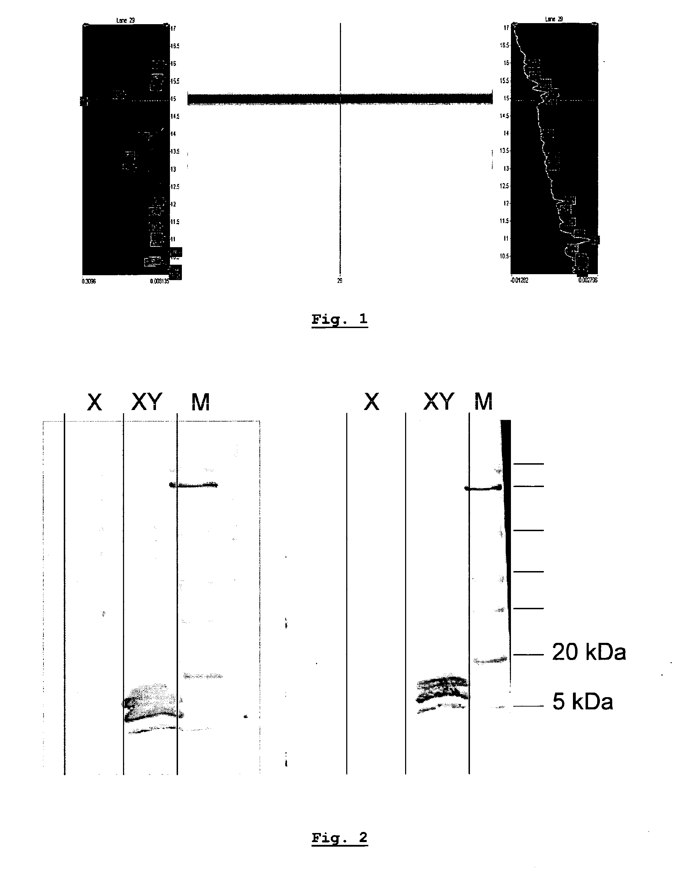

L'analyse du protéome total des spermatozoïdes récolté par exemple chez un bovin pour être ensuite à l'aide d'un vagin artificiel dilué et traité par une cryopréservation dans des paillettes en PUC, a permis d'isoler une fraction protéique (Fraction F) comme étant spécifique des cellules de type Y (figure 1).

Cette fraction protéique contient trois isoformes:
- masses moléculaires respectives de 5, 7 et 10 kDa

### 2. Obtention d'anticorps

La fraction identifiée a été injectée à deux lapins, pour l'obtention d'anticorps.

### 3. Vérification de la spécificité des anticorps

Le sérum de lapin contenant les anticorps polyclonaux a été testé pour sa spécificité: des Western Blot réalisés démontrent clairement que les anticorps polyclonaux sont spécifiques des cellules spermatiques portant le chromosome Y puisque seule la fraction protéique extraite à partir de cellules mâle et femelle à 50/50 réagit avec le sérum.

Le protocole a été réalisé suivant les indications du fournisseur du kit "alkaline phosphatase conjugate substrate kit" 17016432 (BIO-RAD) par une dilution de l'anticorps primaire dans un rapport de 1 : 500.
Des extraits protéiques des cellules non sexées et sexées X sont déposées sur un gel d'électrophorèse acrylamide à 12,5% ; après transfert sur une membrane PVDF de ces extraits cellulaires, la membrane est saturée une nuit à 4°C à l'aide d'une solution TBST (tampon tris salin contenant 0.15% de Tween-20). Après 3 lavages par du TBST, la membrane est mise en présence du sérum de lapin (obtenu par injection de la fraction chromatographique d'intérêt) testé à la dilution adéquate (soit 1/500 éme) par TBST pendant 1h30. Après trois nouveaux lavages par le TBST ,les anticorps « anti-immunoglobulines lapines » de chèvre (par exemple le produit 170-6460 de Biorad) marqués à la phosphatase alkaline sont ajoutés pendant une période de deux heures, après avoir été dilués selon le protocole du fournisseur dans le tampon TBST, trois nouveaux lavages sont réalisés, puis un dernier dans le TBS sans Tween-20 et du phosphate 5-bromo,-4-chloro,-3-indoyle et du nitroblue-tetrazolium sont rajoutés selon les indications du fournisseur (170-6432 de Biorad)jusqu'à obtention du résultat. Un résultat positif correspond à une précipitation du substrat sur la membrane à la position de la protéine d'intérêt.
A la figure 2 on observe 3 bandes protéiniques qui apparaissent uniquement dans l'extrait contenant des cellules porteurs du chromosome X et Y en mélange, tandis que aucune bande n'apparaît dans l'extrait protéique contenant les cellules porteuses uniquement du chromosome X.

### 4. Vérification de la localisation cellulaire de la protéine: protocole d'immunolocalisation sur cellules spermatiques

Le matériel biologique est constitué par les cellules qui sont récoltées et utilisées vivantes. 500 µl de sperme frais sont dilués dans 4 ml de dilueur mis à 37°C. Le sperme est ramené lentement à T° ambiante avant d'être mis à 4°C.

### 5. Immunolocalisation sur cellules vivantes (en tube eppendorf)

- Prélever 300 ul de sperme dilué et les mettre dans un eppendorf stérile;
- Ajouter l'anticorps primaire à 1:50 final ;
- Incuber 4h à une nuit à 4°C, agiter de temps en temps ;
- Centrifuger 2 min. à 600g ;
- Eliminer le surnageant ;
- Mettre 1 ml de dilueur pour rincer et suspendre les cellules ;
- Centrifuger 2 min. à 600g;
- Eliminer le surnageant ;
- Resuspendre les cellules dans 500 ul de dilueur ;
- Ajouter l'anticorps secondaire à 1:50 final ;
- Incuber 1 à 2 heures à T° ambiante ou 1 nuit à 4°C ;
- Centrifuger 2 min. à 600g ;
- Eliminer le surnageant ;
- Ajouter 1 ml de dilueur pour rinçage ;
- Centrifuger 2 min. à 600g ;
- Eliminer le surnageant ;
- Resuspendre le culot dans 300 ml ;
- Déposer 20 ul sur lame ;
- Monter la lamelle ;
- Observer au microscope la fluorescence ;

L'ensemble des cellules d'un échantillon de sperme contenant des cellules de type X et de type Y à proportion de 50/50 n'est pas marqué. Il semble que la réparation du marquage se fasse à raison de 35 à 50% de cellules marquées par l'anticorps.

Après traitement permettant l'accrochage des anticorps, les cellules sont toujours vivantes qu'elles aient été fixées, marquées ou non par l'anticorps.

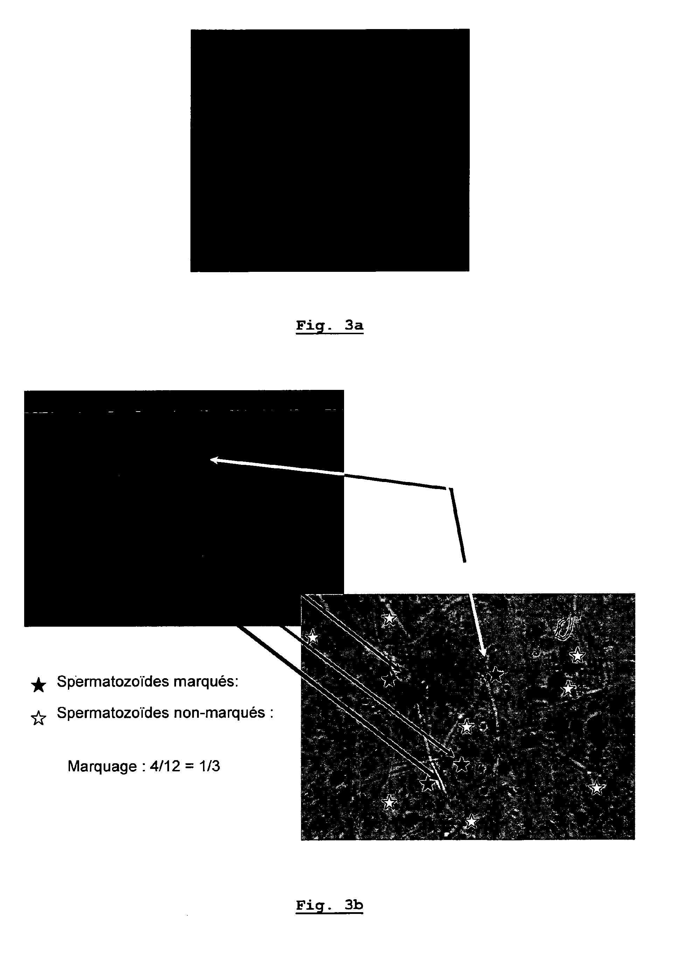

L'anticorps de l'invention est donc spécifique de la surface des cellules de type Y (figure 3).

### 6. Réalisation de sous populations cellulaires

Les anticorps du sérum ont été purifiés afin d'éliminer les protéines du sérum de lapin, en particulier l'albumine.
PROTOCOLE suivant les indications du fournisseur du kit PURE-1A "Protein A Antibody Purification Kit" (SIGMA-ALDRICH).

Des billes magnétiques ont été fixées sur les anticorps purifiés. Ces anticorps fixés aux billes ont été utilisés pour préparer des sous populations de cellules spermatiques sur la base de la spécificité de l'anticorps pour une protéine de surface des spermatozoïdes porteurs du chromosome Y. Le protocole à été réalisé suivant les indications du fournisseur du kit DYNAL, dynabeads M450 epoxy.

### 7. Procédure de génération des deux sous populations

Les cellules sont utilisées le plus rapidement possible après récolte. Elles sont diluées dans un dilueur (à raison de « 20 » million cellules par ml) permettant la conservation de l'intégrité des cellules tout en permettant l'accrochage des anticorps. Les cellules sont mises en contact pendant 30 minutes avec les anticorps soit directement couplés (greffées) aux billes magnétiques (taille comprise entre 50 et 500 nm, de préférence entre 100 et 500 nm) soit indirectement couplées à ces billes par l'intermédiaires d'un second anticorps (anticorps, anti lapin de chèvre) qui fixera des anticorps contenu dans le sérum, cette mise en contact est de 12 à une température comprise entre 4°C et 25°C en agitation douce et à une température comprise entre 4°C et 25°C. Les cellules accrochées aux anticorps munis d'une bille magnétique sont alors soumis à un champ magnétique afin de retenir les cellules de type Y. Le surnageant contenant les cellules X est récupéré. Les cellules spermatiques du surnageant sont alors prêtes pour l'insémination artificielle.

### 8. Vérification du sexe des deux sous population cellulaires caractérisées

Un test de vérification des proportions de cellules de chaque sexe dans les deux populations cellulaires (fixées et non fixées sur les billes) est réalisé. Il consiste en l'amplification d'un gène spécifique soit du chromosome X soit du chromosome Y. Ce test de vérification de la constitution des deux populations permet de comparer les proportions de chacun des types de cellules dans les deux populations.

La théorie veut que, à la vue des observations réalisées, la population retenue, prétendument les cellules de type Y, ne contienne que des cellules portant le chromosome Y. Cependant, la population cellulaire contenue dans le surnageant, prétendument les cellules de type X, est représentée majoritairement par des cellules de type X, mais contient un taux de cellules portant le chromosome Y. En effet, il semble, à la vue des observations d'immunolocalisation in situ, que tous les spermatozoïdes de type Y ne portent pas l'antigène à leur surface. Les observations montrent que la structure d'intérêt est présente au niveau de la tête et de la deuxième moitié du flagelle de spermatozoïde.

La population cellulaire contenue dans le surnageant a été utilisée pour des inséminations artificielles. Le sexage des embryons obtenu donne les résultats suivant en termes de sex-ratio : (en cours).

### 9. Procédé d'insémination des spermatozoïdes chez les bovins

Les cellules sélectionnées par le procédé de l'invention sont conservés dans des paillettes en PUC à paroi fine dans de l'azote liquide à - 196°.

Les paillettes sont introduites dans un pistolet (tube métallique recouvert de plastique à usage unique) et comportant un piston apte à éjecter le liquide contenu dans les paillettes. Le pistolet permet par une manipulation manuelle, l'injection des cellules dans le col de l'utérus à la base des deux cornes utérines.

## Revendications

1. Une structure antigénique de surface des cellules spermatiques associées au chromosome Y et présentant un poids moléculaire compris entre 5 kDa et 12 kDa.

2. La structure antigénique selon la revendication 1, **caractérisée en ce qu'**elle présente un poids moléculaire de 5 kDa, 7 kDa ou 10 kDa.

3. Une molécule dirigée spécifiquement contre la structure antigénique selon la revendication 1 ou la revendication 2.

4. La molécule selon la revendication 3, **caractérisée en ce qu'**elle est un anticorps ou une portion hypervariable d'un anticorps.

5. La molécule selon la revendication 4, **caractérisé en ce qu'**elle est un anticorps monoclonal.

6. Une trousse de sexage comprenant la molécule selon l'une quelconque des revendications précédentes, 3 à 5 et éventuellement la structure antigénique selon la revendication 1 ou 2.

7. Un support solide comprenant la molécule selon l'une quelconque des revendications précédentes 3 à 5, fixé sur la surface dudit support solide.

8. Le support solide selon la revendication 7 **caractérisé en ce qu'**il est constitué de billes, de préférence de billes magnétiques.

9. Le support solide selon la revendication 8 **caractérisé en** se qu'il est constitué de billes aillant un diamètre compris entre 50 mm et 9 µm, de préférence compris entre 100 mm et 400 nm.

10. Un procédé de discrimination entre des cellules spermatiques portant le chromosome Y et des cellules spermatiques portant le chromosome X, **caractérisé en ce qu'**il comprend les étapes suivantes:
- une mise en contact desdites cellules, avec une molécule selon l'une quelconque des revendications précédentes 3 à 5 ou le support solide selon l'une quelconque des revendications 7 à 9, dans des conditions aptes à obtenir une entre lesdites molécules ou ledit support solide et la structure antigénique de la revendication 1 ou 2 ;
- une liaison ou marquage des cellules portant le chromosome Y par l'interaction entre lesdites molécules et ladite structure antigénique ;
- éventuellement une séparation entre les molécules marquées ou liées par lesdites molécules et les cellules non marquées ou non liées par lesdites molécules ;
- éventuellement recueillement des cellules marquées ou liées ou des cellules non marqués et non liées.
